# EUROPEAN PATENT SPECIFICATION

(11) **EP 0 917 563 B1**
(45) Date of publication and mention of the grant of the patent: **02.02.2005**
(21) Application number: 97933797.9
(22) Date of filing: 30.07.1997
(51) Int. Cl.: C12N 1/20, C12N 15/52, C10G 32/00, C12P 17/04

(54) **MICRO-ORGANISM WHICH CAN DESULPHURISE BENZOTHIOPHENES**
MIKROORGANISMUS DER BENZOTHIOPHENE ENTSCHWEFELN KANN
MICRO-ORGANISME POUVANT DESULFURER DES BENZOTHIOPHENES

(30) Priority: 30.07.1996 GB 9615928
(43) Date of publication of application: 26.05.1999
(73) Proprietor: The Court of Napier University, Edinburgh EH10 5DT (GB)
(72) Inventor: OLDFIELD, Christopher, East Lothian EH41 3SD (GB)
(74) Representative: Pattullo, Norman
(86) International application number: PCT/GB1997/002055
(87) International publication number: WO 1998/004678

(56) References cited:
- EP-A- 0 441 462
- US-A- 4 562 156
- KIM, BYUNG HONG ET AL: "Selectivity of desulfurization activity of Desulfovibrio desulfuricans M6 on different petroleum products" FUEL PROCESS. TECHNOL. (1995), 43(1), 87-94 CODEN: FPTEDY;ISSN: 0378-3820, 1995, XP002050488
- SAGARDIA, FERMIN ET AL: "Degradation of benzothiophene and related compounds by a soil Pseudomonas in an oil-aqueous environment" APPL. MICROBIOL. (1975), 29(6), 722-5 CODEN: APMBAY, 1975, XP002050489

## Description

The present invention relates to the desulphurisation of petrochemicals. More particularly the invention relates to micro-organisms which can break down benzothiophenes, a process for isolating the micro-organisms and the use of these organisms in the desulphurisation of petrochemicals.

Crude oil and its distillates contain significant amounts of organic sulphur in the form of alkyl- and cycloalkyl thiols, alkyl- and aryl thioethers, and aromatic compounds based on thiophene. Combustion of these compounds results in the liberation of sulphur oxyacids (SOₓ) into the atmosphere, and hence to acid rain. Atmospheric SOₓ is a major contributing factor to poor air-quality in the city environment; acid rain is a primary cause of deforestation, since it lowers the soil pH to levels which are intolerable for many trees and plants.

In order to combat the problems caused by atmospheric SOₓ, legislation has been implemented world-wide to drive a progressive reduction in the sulphur content of fuels. European Community legislation requires a maximum limit of 0.05% wt for on-road diesel as of 1 October 1996. The sulphur content of gas-oil and middle distillates (from which heating fuels are derived) has been set at maximum value of 0.2% wt as of 1 October 1994, with legislation for further reductions pending.

Sulphur in crude oil also causes problems for refiners since it poisons metal catalysts used in the refinery process. For this reason refinery streams are routinely treated in order to lower the sulphur content. In this process hydrodesulphurisation (HDS), organosulphur undergoes catalytic hydrogenation, resulting in the release of sulphur as H₂S. In its conventional specification (catalyst type, operating temperature and pressure) HDS removes about 70% of the sulphur. The desulphurisation efficiency varies according to the type of compound, decreasing in the order: aliphatic»cycloalkyl»aromatic. Thus the residual organosulphur left after conventional HDS treatment is mainly aromatic (thiophenic) sulphur.

Legislation targets for sulphur levels set for various refinery products have previously been met by a judicious choice of crude oil in combination with blending (reformulation) of HDS-treated refinery streams. However the increasing stringency of legislation, coupled with the gradual exhaustion of low-sulphur reserves, means that it is becoming increasingly difficult for refiners to meet these targets. In principle, these targets can be met by increasing the efficiency of HDS. Thus, doubling the operating pressure from ca.30bar (standard HDS plant) to ca.60bar permits an increase in desulphurisation efficiency from 75% to 97%. However, the cost to the refiner of installing and running the plant necessary to operate the modified HDS is high. CONCAWE (the Oil Companies' European Organisation for Environment, Health and Safety) estimates that the combined cost to refiners within the 12 member states of the EU will be 3 × 10⁹ US$. This consideration alone has led to a market-opportunity for alternative, less costly, options for achieving the requisite levels of desulphurisation.

one such approach is the use of biocatalysts (microbes or their constituent enzymes) which specifically degrade organosulphur compounds. Microbial desulphurisation (MDS) offers the advantages normally associated with biotechnological process, viz. high specificity for the target compounds, safe operation at relatively low temperatures and pressures (hence no need for sophisticated plant) and inherently non-pollutive.

MDS is not a new concept, the possibility of using bacteria to remove inorganic sulphide from coal was first suggested 30 years ago. Since then much has been written concerning the microbiological, engineering and economic aspect of this kind of technology. Nevertheless the development of MDS remains circumscribed at the present time by a lack of organisms capable of carrying out relevant desulphurisation reactions.

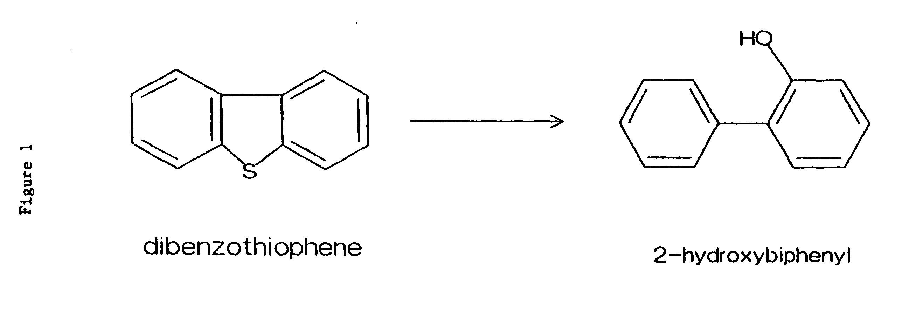

Interest in the application of microorganisms as a convenient and economical route to the removal of organic sulphur compounds from fossil fuels led to the recent discovery of *Rhodococcus* sp. strain IGTS8 as disclosed in US Patent No. 5,104,801. Strain IGTS8 is able to "desulphurise" dibenzothiophene (DBT) to 2-hydroxbiphenyl (HBP) and inorganic sulphite (Fig. 1). DBT is widely regarded as a model compound, representative of the aromatic organosulphur fraction of coal and crude oil. This is an interesting reaction for many reasons, not least because strain IGTS8 cannot metabolise HBP. Thus, when strain IGTS8 is grown in media containing DBT as the sole source of sulphur, DBT serves as a source of sulphur for biomass and HBP simply accumulates in the medium (Kayer et al 1993; Oldfield et al, 1997). Therefore this desulphurisation reaction is regarded as representative of a new class of secondary metabolic pathway, carrying out a responsible for sulphur-scavenging reaction.

The strain IGTS8 desulphurisation pathway has been the subject of considerable study. The DBT-desulphurisation phenotype is conferred by the plasmid-located dsz operon which encodes three proteins, Dsz A, B and C, which are necessary and sufficient for DBT desulphurisation. The operon has been cloned and sequenced (Denome et al, 1993, 1994), and the metabolic pathway has been elucidated (Olson et al, 1993; Oldfield et al, 1997). The enzymes have been purified and preliminary characterisation studies have been published (Lei & Tu, 1996; Gray et al, 1997). Genetic analysis of the regulatory region of the dsz operon indicates that the primary regulatory circuit is repression by more readily biovailable sulphur (sulphate, cysteine, methionine) (Li et al, 1996; Oshiro et al, 1996b), a result which is consistent with the hypothesis that this pathway serves a sulphur-scavenging function.

Since the isolation of stain IGTS8 was reported, several other research groups have reported the isolation of DBT-desulphurising bacteria by enrichment culture using mineral salts media containing DBT as the sole sulphur source.

These include *Rhodococcus* sp. strain SY1 (Omori et al, 1995); first reported as Corynebacterium sp. strain SY1 (Omori et al, 1992), *Rhodococcus erythropolis* strain D-1 (Izumi et al, 1994), *R. erythropolis* strain H-2 (Ohshiro, Suzuki and Izumi, 1996a), R. *erythropolis* strain N1-36 (Wang et al, 1996a, b) and strain ECRD-1, initially classified as a strain of *Arthrobacter* sp. (Lee, Senius and Grossman, 1996), but more recently confirmed as a strain of *Rhodococcus* sp. (Denis-Larose et al, 1997). Ten DBT-desulphurising strains ahve been isolated by the Applicant's laboratory from British soils and all of these are rhodococci (S.C. Gilbert, J. Morton and C. Oldfield, unpublished work). There is no evidence that the desulphurising properties of any of these strains are different from that of strain IGTS8, and these strains are best regarded as re-isolations of strain IGTS8. Therefore it is reasonable to conclude that the DBT-desulphurisation phenotype is confined exclusively within the genus *Rhodococcus.*

The DBT-desulphurisation enzymes have a fairly relaxed specificity for members of the DBT family (ie molecules which can be regarded as deriviates of dibenzothiophene generated by alkyl or aryl substitution at different points on the parent molecule, and desulphurisation always results in the formation of the corresponding monophenol (Lee, Senius and Grossman, 1995; Ohshiro, Suzuki and Izumi, 1996a).

Despite their broad specificity for derivatives of DBT, DBT desulphurisers (ie organisms isolated by enrichment culture using DBT as sole sulphur source) seem to be largely incapable of desulphurising benzothiophene (BTH). For example, the desulphurisation activity of strains ECRD-1 and N1-36 towards BTH are negligible (Lee, Senius and Grossman, 1996; Wang and Krawiec, 1996a). None of the ten isolates obtained by the Applicant are capable of desulphurising DBT.

IGTS8 has a number of properties which make it particularly attractive for the purpose of desulphurisation, not the least of which is that the sulphur is released as water-soluble sulphate, and that the organism cannot degrade hydrocarbons, and so cannot degrade the oil itself.

IGTS8 desulphurises DBT, but not BT or thiopene (T), or any other compound relevant to fossil fuel desulphurisation, furthermore its ability to degrade alkyl-substituted dibenzothiophenes, which typically account for most of the dibenzothiophenic sulphur in oil, is considerably poorer than its ability to degrade DBT itself.

It is an object of the present invention to provide a micro-organism which is capable of desulphurising organosulphur compounds.

In one embodiment the invention provides isolate NUE213E deposited under Accession No NCIMB40816 at The National Collections of Industrial and Marine Bacteria Limited, 23 St Machar Drive, Aberdeen, AB2 1RY on 29 July 1996.

In an alternative embodiment the invention provides isolate NUE213F deposited under Accession No NCIMB 40817 at the National Collections of Industrial and Marine Bacterial Limited, 23 St Machar Drive, Aberdeen, AB2 1RY on 29 July 1996.

Preferably the invention provides a micro-organism which can desulphurise both benzothiophenes and dibenzothiopenes.

Typically the micro-organism is rod-shaped and stains Gram-positive.

The invention further provides a process for the isolation of an organism as previously defined, the process comprising the step of isolating the organism by selective enrichment of micro-organisms capable of utilising benzothiophene as a sole source of sulphur for growth.

Preferably the process comprises the steps of obtaining soil samples from the base of an oil shale spoil heap, suspending soil in minerals medium with benzothiophene as sole source of sulphur, repeatedly passaging organisms in the soil in minerals medium containing benzothiophene as sole source of sulphur, streaking cultures on agar plates to obtain isolated colonies of micro-organisms capable of utilising benzothiophene as a sole source of sulphur for growth and preparing cultures from individual colonies.

Typically the micro-organisms produced according to the above process accumulates large amounts of phenol and growth medium.

This invention has important implications for the development of microbial petrochemical desulphurisatic technologies since a considerable fraction of the organosulphur in petrochemicals is represented by benzothiophenes (BTHs).

The following experimental evidence describes the isolation and preliminary characterisation of *Rhodococcus* sp. Strain 213E and 213F organisms which efficiently desulphurise BTH to a phenol, tentatively-identified as 2-[z-2'hydroxythenyl] phenol (HEP). Characterisation of BTH metabolites recovered from strain 213E culture media has permitted the outline characterisation of the metabolic pathway for BTH desulphurisation and this is compared with the detailed pathway for DBT desulphurisation (Oldfield et al, 1997 in press). Strain 213E is unable to desulphurise DBT, and strain IGTS8 is likewise unable to desulphurise BTH. The reasons for this are discussed in terms of the biochemistry of the desulphurisation reactions. Strain 213F is able to desulphurise DBT and BTH.

The invention is illustrated with reference to the accompanying figures wherein:
**Figure 1** illustrates the overall desulphurisation reaction carried out by strain IGTS8.
**Figure 2** illustrates growth curves for (a) Strain 213E grown in FruRM/BTH; (b) Strain 213F grown in FruRM/BTH (c) Strain 213F grown in FruRM/DBT; (d) strain IGTS8 grown in FruRM/DBT.
**Figure 3** illustrates gas-chromatographic spectrum of ethyl acetate extract of culture medium of strain 213E grown in FruRM/BTH to end of exponential growth phase. **See Table 3** for peak assignments.
**Figure 4** illustrates mass spectra of BTH metabolites identified by Gas-chromatography.
**Figure 5** illustrates the proposed metabolic pathway for BTH desulphurisation. The route is BTH → BTHO → BTHO₂ → HBESi → HEP, with spontaneous cyclisation of HEP to BFU. HBESi⁻ was isolated as its condensed form, phenylene sultine (PSi), and the interconversion is included in the Fig.
**Figure 6** illustrates the overall desulphurisation reaction carried out by strains 213E and 213F.
**Figure 7** illustrates a proposed mechanism for the final desulphination step (HBESi⁻ → HEP), in the BTH desulphurisation pathway. The sulphur product is predicted to be sulphite and this has to be confirmed by experiment.
**Figure 8** (reproduced from Figure 4 Oldfield et al 1997 in press) illustrates the metabolic pathway for DBT desulphurisation by IGTS8.
Figure 9 (reproduced from Figure 6 Oldfield et al 1997 in press) illustrates the pathway for desulphurisation of DBT to DHBP.

### METHODS

Materials Sulphite oxidase (suspension in 2.3 M (NH₄)₂SO₄), bovine-heart cytochrome c, the buffers HEPES and HEPPS and A.C.S-grade sodium sulphite, Sigma (Poole, Dorset, UK); DBT (99+%), DBTO₂ (97%), HBP (sold as 2-phenylphenol; 99+%), BTH (sold as thianaphthene; 99%) and BFU (sold as 2,3-Benzofuran, 99.%), Aldrich (Dorset, UK); Bacto and Noble agar (Difco, Surrey, UK); Glucose, fructose and glycerol (Merck Ltd, Dorset, UK). Glass distilled water was used throughout.

**Bacteria** *Rhodococcus* sp. strain IGTS8 (ATCC 53968) was obtained from J. Kilbane (Institute of Gas Technology, Chicago, I11 USA). *Rhodococcus sp.* strain 213E (NCIMB 40816) was isolated as set out below. Strains IGTS8 and 213E were maintained for routine use on SRM agar plates (Difco Noble agar, 20 g/l) containing 200 µM DBT or 200 µM BTH, respectively, and were sub-cultured every week.

Culture Conditions SRM has the following composition (per litre): Na₂HPO₄, 4.33g; KH₂PO₄, 2.65g; glucose, 20g; NH₄Cl, 2g; MgCl₂. 6H₂O, 0.64g; nitrilotriacetic acid, 0.1g; CaCl₂.2H₂O, 33 mg; ZnCl₂, 2.6mg; FeCl₂.4H₂O, 2.6mg; EDTA, 1.25mg; MnCl₂.4H₂O, 1.0mg; CuCl₂.2H₂O, 0.15 mg; Co(NO₃)₂.6H₂O, 0.125 mg; Na₂B₄O₇-10H₂O, 0.10mg; (NH₄)₆Mo₇O₂₄4H₂O, 0.09mg. Glucose was added as a filter-sterilised solution following autoclaving (121°C, 20 min). The final pH was 7.2 without titration. In some experiments another sugar replaced glucose as the carbon-source. Thus Fructose-Rhodococcus Medium (FruRM) contained fructose (10g/l) and Glycerol-Rhodococcus Medium (GlyRM) contained glycerol (10.22g/l).

For cultures of strain IGTS8, DBT (200 *µ*M, added from a stock solution, 40 mM in acetone) was included. For cultures of strain 213E, BTH (200 *µ*M, added from a stock solution, 40 mM in acetone) was included. Sodium sulphate (200 *µ*M) was used as a sulphur source for cultures of IGTS8 and 213E expressing no desulphurising activity.

Isoation of Rhodococcus sp. strains 213E and 213F Rhodococcus sp. strains 213E and 213F were isolated by enrichment culture on GlyRM/BTH (benzothiophene as sole source of sulphur and glycerol as carbon source), using as an inoculum a soil sample taken from the vicinity of a oil shale spoil heap located at a disused Scottish mine. 100 ml GlyRM/BTH in a 250 ml Ehrlenmyer flask was inoculated with 20 g soil and incubated in an orbital shaker (120 rpm, 30°C) for 14 days. 1 ml of this culture was used to inoculate a further 50 ml GlyRM/BTH in a 100 ml Ehrlenmyer flask and incubated under the same conditions for 14 days. This procedure was repeated a further four times. 100 *µ*l aliquots from the fifth transfer were serially diluted onto GlyRM/BTH plates (15g/l Noble Agar) and these were incubated at 30°C until average colony sizes of 0.5-5mm were obtained. At this stage several different organisms could be discerned, based on colony morphology and Gramstaining properties. Representative single colonies of each were picked and used to inoculate 20 ml aliquots of GlyRM/BTH in 50 ml Ehrlenmyer flasks. The flasks were incubated (120 rpm, 30°C) until O.D.₆₀₀ > 9.0, approx. (about 5 days). Flasks inoculated with colonies designated 213E and 213F, only, yielded significant growth in the liquid medium at this stage. Aliquots of each culture were centrifuged (12000 rpm for 5 min), and 10 µl of a solution of Gibbs reagent (10 mM in acetone) was added to 1 ml of supernatant. In both cases a blue colour developed within a few minutes, indicating the presence of phenolic compounds. Serial dilution and plating out of 213E and 213F at 5 days revealed predominantly one organism in each case, as judged by colony morphology and Gram staining. Sleeted colonies were grown up in GlyRM/BTH medium and a further round of plating yielded only one organism by the same criteria. The two isolates were referred to as strains 213E and 213F.

**Growth studies SRM** (50ml in a 100ml Erlenmeyer flask) was inoculated with a loopful of cells from a plate and incubated in an orbital shaker (250 rpm, 30°C). At intervals as appropriate, an 3ml sample was removed aseptically for measurement of biomass (as optical density at 600 mm, O.D.₆₀₀) and for phenol production using the Gibbs Assay. The Gibbs Assay was carried out as follows: 1.5ml of the sample was aliquoted into a Eppendorf tube, centrifuged (12000 rpm, 5 min) to remove the cells and frozen (-20°C) until the end of the experiment. The samples were then thawed out and 1.0ml of the supernatant was transferred to a 1ml disposable spectrophotometer cuvette. 10 *µ*l Gibbs reagent (10mM in acetone) was then added to each cuvette. A blank solution (SRM + 10µl Gibbs reagent) was also prepared. The set was incubated overnight at 30°C, for full colour development, and the absorbance at 610nm was measured.

Measurement of specific desulphurisation activity Cells were grown to the end of log-phase (about 60 h) and recovered by centrifugation (15,000 x g for 15 min), washed twice by re-suspension in 10 volumes of 50 mM HEPPS buffer, pH 8.0 and finally re-suspended in the same buffer to a density of approximately 250 A₆₀₀ (1cm path-length cell). The suspension was kept on ice and used on the day of harvesting. Specific activities were measured in terms of phenol production, using the Gibbs assay, or of sulphite production, using the sulphite oxidase assay, as described below.

**Standard Gibbs Assay for HBP** The stock 250 A₆₀₀ cell-suspension was diluted with 50 mM HEPPS, pH 8.0, to a nominal A_{*600*}=1.0. This was done by diluting the suspension to give a spectrophotometer reading of 0.1-0.2 A₆₀₀, and calculating the dilution factor required to obtain 1.0 A_{*600*}. For both strain IGTS8 and strain 213E, 1.0 A_{*600*} corresponds to a cell density of 0.35g dry weight/1. Substrate (25 *µ*M final concentration, introduced from a 40mM stock in acetone) was added to 40 ml of freshly diluted 1.0 A_{*600*} cell-suspension in a 100ml stoppered Ehrlenmeyer flask and incubated in an orbital shaker (240 rpm., 30°C) for 60 min. At 10 min intervals 1.5ml aliquots were removed to Eppendorf tubes and centrifuged (12,000 rpm, 5 min) to remove cells. 1.0 ml of the supernatant was transferred to a 1 ml disposable spectrophotometer cuvette and stored at 4°C until the end of the incubation. 10µl Gibbs reagent (10 mM in acetone) was then added to each cuvette. A blank solution (HEPPS buffer pH 8.0 + 10 µl Gibbs reagent ) was also prepared. The set was incubated overnight at 30°C, for full colour development, and the absorbance at 610mm was measured. For assays of DBT desulphurisation (to HBP), the ▲A_{*600*} was converted to HBP concentration using a standard curve prepared with authentic HBP in the range 0-30 µM. The linear regression slope of a plot of [HBP] vs. time gave the reaction rate (µM/h); the specific activity (*µ*mol g⁻¹ h⁻¹) was obtained by dividing the rate by the cell concentration (g dry weight 1⁻¹). For assays of BTH desulphurisation the linear regression slope of a plot of A₆₀₀ vs. time was divided by 3.7 × 10³ M⁻¹cm⁻¹, this being an estimate of the extinction coefficient of the phenol-Gibbs adduct obtained from growth curve data (see results). This molar rate of phenol production was divided by the cell concentration (g dry weight 1⁻¹) to give a specific activity.

Isolation of organic metabolites and GC-MS analysis Cultures of strain IGTS8 or strain 213E were grown to mid-log phase (about 36 h) or end log-phase (about 60h). Cells were removed by centrifugation at 2800 x g (3000 rpm) for 30 mins and the supernatant was titrated to pH 1 with 50% HCl and extracted three times with equal volumes of ethyl acetate. The ethyl acetate extracts were pooled and dried by stirring for 1 h with anhydrous MgSO₄ (100 g/l). The ethyl acetate was removed by rotory evaporation and the solids redissolved in 3.0 ml ethyl acetate.

GC-MS analysis of benzothiophene metabolites was performed on a Hewlett Packard 5890 series II gas chromatograph coupled to a 5972 series mass selective detector. The GC was fitted with a Hewlett Packard capillary column HP-5MS, of length 30m. The carrier gas was helium. Run conditions were as follows: Start temperature 40°C held for 2 min, followed by 10°C/min ramp rate to final temperature 240°C. Total run time, 24 min. The mass scan range was 40 to 550 amu.

### RESULTS

*Rhodococcus* sp. strains 213E and 213F were and isolated by enrichment culture from an old oil-shale spoil heap In setting up the enrichment procedures which led to the isolation of strains 213E and 213F, a variety soilds, water and marine sediments, contaminated with oil or coal, either through natural process or human action, were used as inocula. Using the enrichment medium GlyRM, two organisms, strains 213E and 213F, were isolated which were capable of growing on BTH as sole sulphur source.

Strain 213E was isolated from soil samples taken from the foot of an oil-shale spoil heap located at a disused mine. When grown on SRM/BTH Noble Agar plates, colonies of strain 213E were pink with entire margins and umbonate elevation. The organism grew in SRM/BTH (glucose as sole carbon source, BTH as sole sulphur source). Other carbon sources, notably fructose, mannitol, sodium pyruvate and tri-sodium citrate, and of course glycerol, could be substituted for glucose in this medium. Strains 213E grew in FruRM with faster doubling times than recorded for any other carbon source (data not shown). Sulphate could be substituted for BTH, but there was no growth when DBT was substituted for BTH. Strain IGTS8 grew equally well in FruRM and SRM (data not shown).

In FruRM/sulphate and FruRM/BTH media strains 213E and 213F both stained as Gram +ve rods of irregular length. Both organisms were found to be non-motile, partially acid-fast, catalase positive, oxidase positive with strictly aerobic growth. The strains have been identified as belonging to the genus *Rhodococcus* (M. Goodfellow, University of Newcastle-upon-Tyne, personal communication). More complete characterisation is in progress.

### Rhodococcus sp. strains 213E and 213F grew in mineral salts medium using BTH as a sole source of sulphur

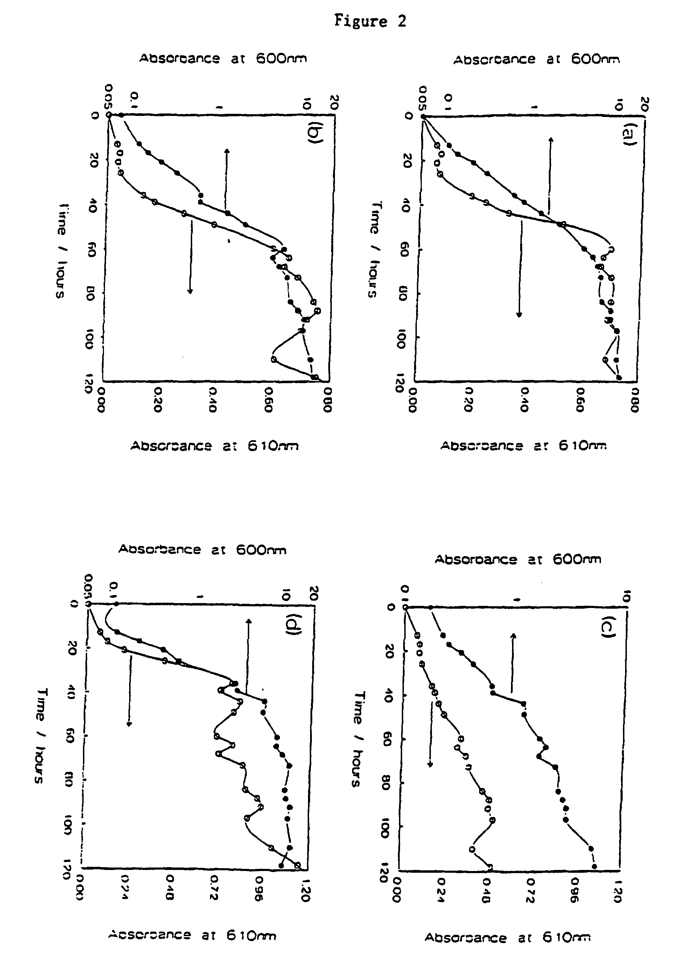

Cells from a single colony of *Rhodococcus* sp. strain 213E, or of strain 213F, taken from a FruRM/BTH agar plate, was used to inoculate 50ml of SRM containing either BTH (200 *µ*M) or sulphate (200 *µ*M) in a 100ml Ehrlenmyer flask. similarly, cells from a single colony of *R. erythropolis* strain IGTS8, taken from an SRM-agar plate containing DBT as sole sulphur source, was used to inoculate 50 ml of SRM containing either DBT (200 *µ*M) or sulphate (200 *µ*M). The flasks were incubated in an orbital shaker (120 rpm, 30°C). At intervals 3ml was withdrawn from each culture under sterile conditions for measurement of biomass and phenol concentration, as described in methods. The cells were also Gram stained and examined under the microscope. Typical growth curves are shown in Fig 2 and mean generation times (MGTs) are given in Table 1. It can be seen from the table that MGTs in FruRM/sulphate medium are approximately the same for all three strains (around 4h). The MGT in FruRM with the appropriate organosulphur source (DBT for strain IGTS8, BTH for strains 213E and 213F), was approximately the same (around 8 h). Strain 213F grew in FruRM/DBT with MGT 12h (Table 1). There was no significant growth of strain IGTS8 or of strain 213E in FruRM -only (i.e. no added sulphur compound), over a period of 100 h. There was no significant growth of strain 213E in FruRM/DBT, or of strain IGTS8 in SRM/BTH, over the same period.

Strain IGTS8 stained as a Gram +ve coccobacillus when grown in both SRM/sulphate and SRM/DBT. Strains 213E and 213F stained as Gram +ve rods of irregular length when grown in both FruRM/sulphate and FruRM/BTH. In none of the cultures were age-dependent changes in size, shape of Gram-staining properties observed between onset and end of exponential growth.

During growth of strain IGTS8 in SRM/DBT (but not in SRM/sulphate) a phenolic compound accumulated, as detected using the Gibbs phenol assay, as expected on the basis of earlier studies (Kayser et.al, 1993)). GC-MS analysis of ethyl acetate extracts of culture supernatants, recovered at end log-phase, yielded a major peak with GC retention time of (14.08 min) and mass-spectrum identical to that of authentic HBP (data not shown). Therefore it was concluded that strain IGTS8 desulphurised DBT to HBP, as expected (Olson et al, 1993; Oldfield et al, 1997). No phenol was detectable in the supernatant of cultures of strain IGTS8 grown on SRM/sulphate, as measured using Gibbs reagent.

A phenolic compound similarly accumulated during growth of strain 213E in SRM/BTH medium. This compound was likewise assumed to be the product of BTH desulphurisation. As discussed below, this identity was assigned to the 10.9 min peak observed in the GC spectra of ethyl acetate extracts of SRM/BTH culture media. The mass spectrum of this peak was consistent with its identity as 2-[z-2'hydroxyethenyl] phenol (HEP), as discussed below. No phenol was detectable in the supernatant of cultures of strain 213E grown on SRM/sulphate, as measured using the Gibbs reagent.

Strain 213E grew in SRM/BTH with MGT 9h. In order to test the hypothesis that the longer MGT was the result of a toxic effect of BTH, growth curves were obtained for a series of BTH concentrations in the range 50-200 *µ*M. The MGT was found to be independent of the BTH concentration (Table 2). It was therefore concluded that BTH was not toxic to strain 213E in the concentration range <200 *µ*M. It can also be seen from Table 2 that the biomass increases with increasing BTH concentration, indicating that this was a sulphur-limited medium.

Metabolites extracted from culture supernatants of strain 213E grown in SRM/BT enabled the elucidation of a metabolic pathway for BTH desulphurisation

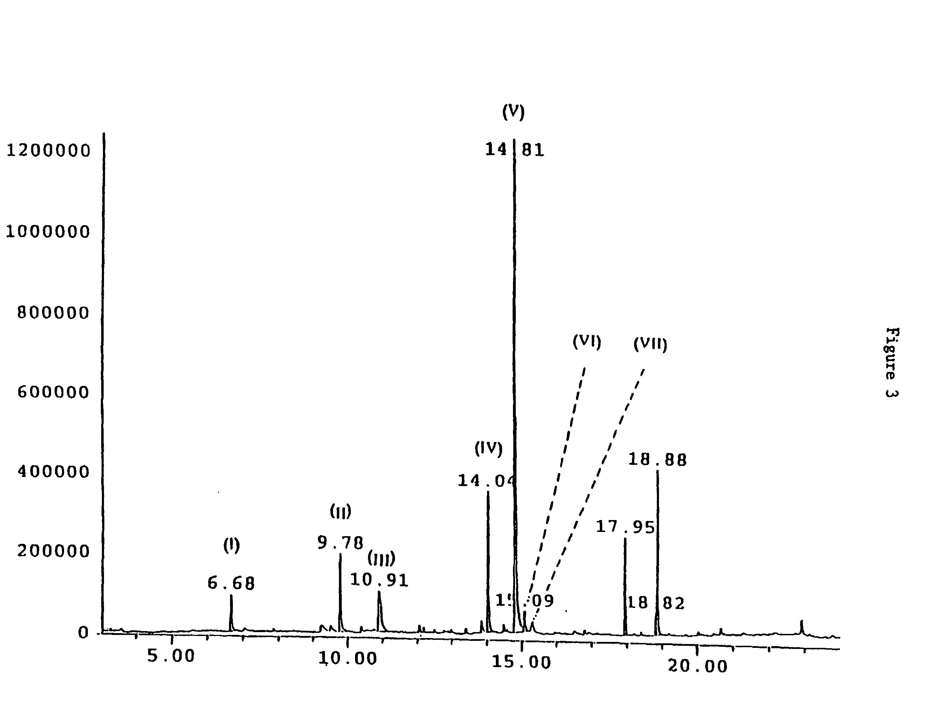

Cultures of strain 213E in FruRM/BTH or FruRM/sulphate were grown to the end of exphonential growth phase. The cells were removed by centrifugation and the supernatant was acidified and extracted with ethyl acetate, as described in Methods. Gas-chromatographic analysis of ethyl acetate extracts (Fig 3) from FruRM/BTH cultures revealed a total of seven unique peaks (i.e. peaks which were absent from extracts of FruRM/sulphate cultures). These peaks were labelled (I)-(VII) in order of increasing elution time. Mass-spectroscopic analysis of each peak enabled their identification as summarised in Table 3. other peak (not numbered) correspond to compounds which are not regarded as BTH metabolites; they also appear in ethyl acetate extracts of cells grown on FruRM/sulphate. The 17.95 min peak is a substituted benzene 1,2 dicarboxylate and the 18.88 min peak is hexadecanoate (data not shown).

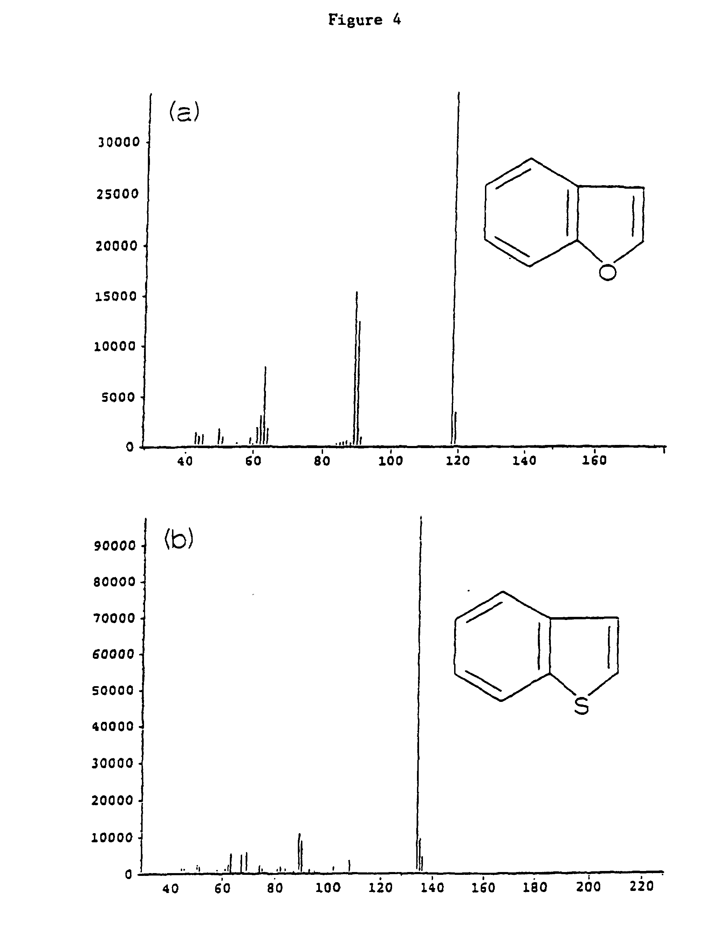

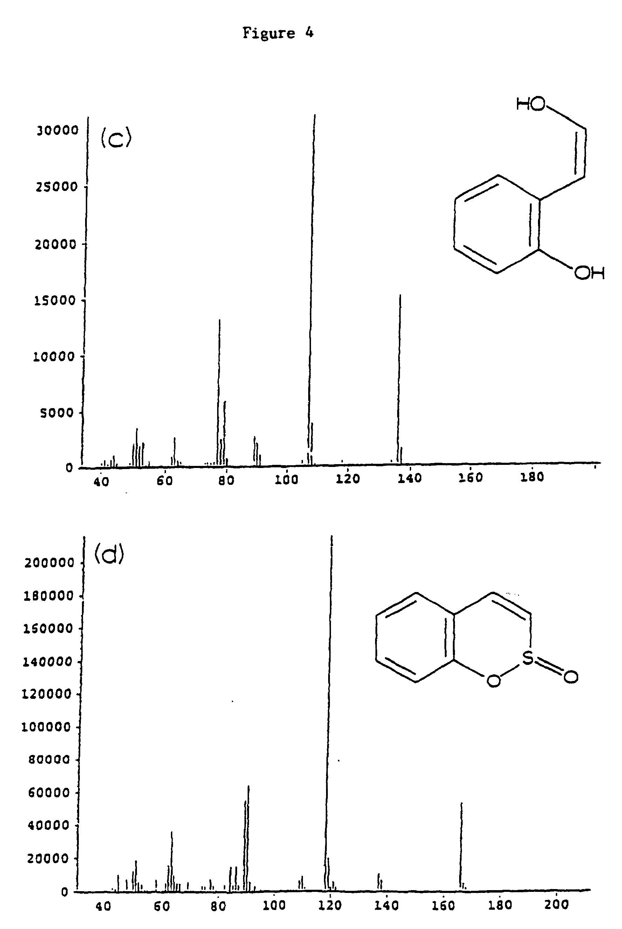

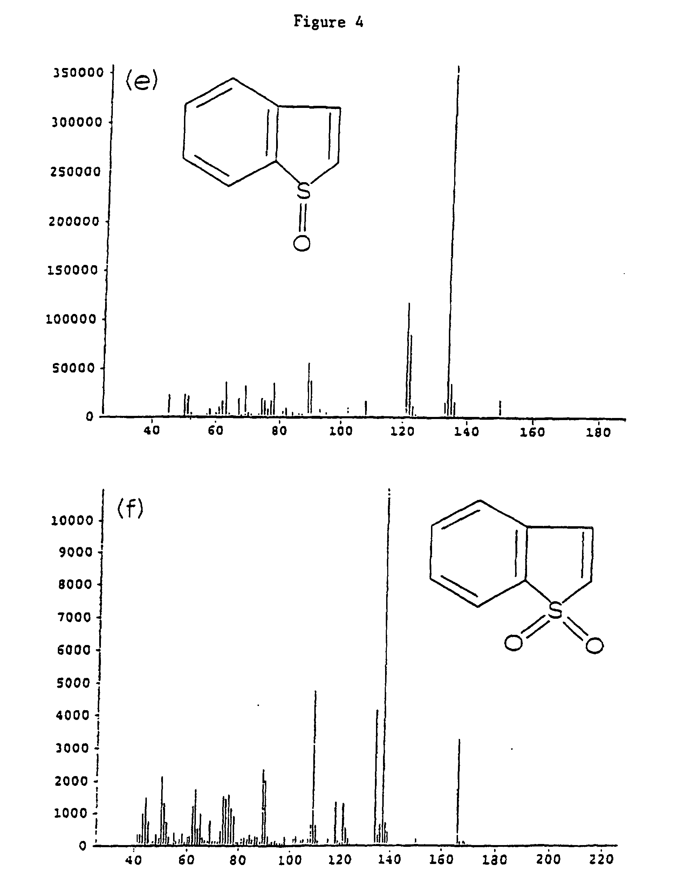

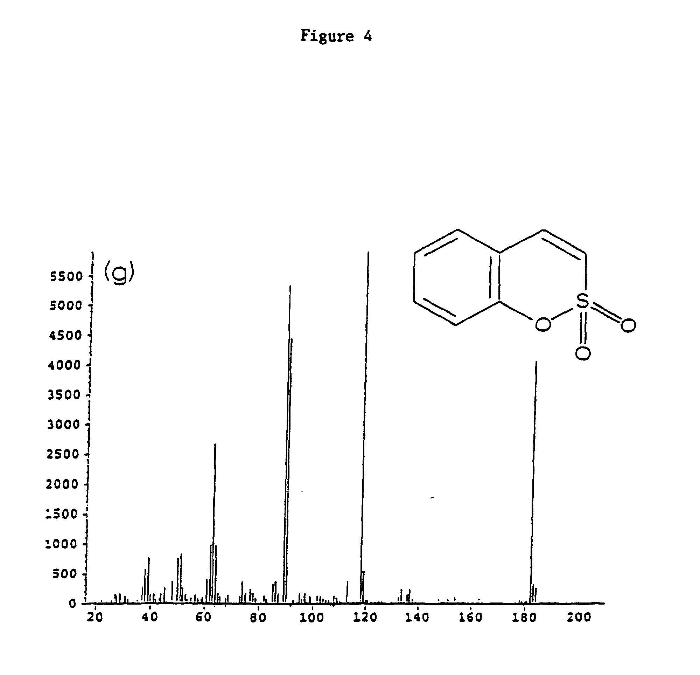

Mass spectra of the peaks (I)-(VII) are shown in Figure 4. Peaks (I) and (II) were identified as benzofuran (BFU) and benzothiophene (BTH) respectively (Fig 4(a) and (b)). The GC retention times and mass-spectra were identical to those of commercially available standards. In some experiments the ethyl acetate extract was spiked with either BFU or BTH standards, and these eluted with retention times coincident with peak (I) and (II), respectively. It was concluded that BTH was residual unconverted substrate and that BFU was a product of the desulphurisation reaction, as discussed below.

The remaining peak (III-VII) were assigned on the basis of their mass spectra, as shown in Fig 4 (b)-(g). See legend to Fig 4 for details of the assignments.

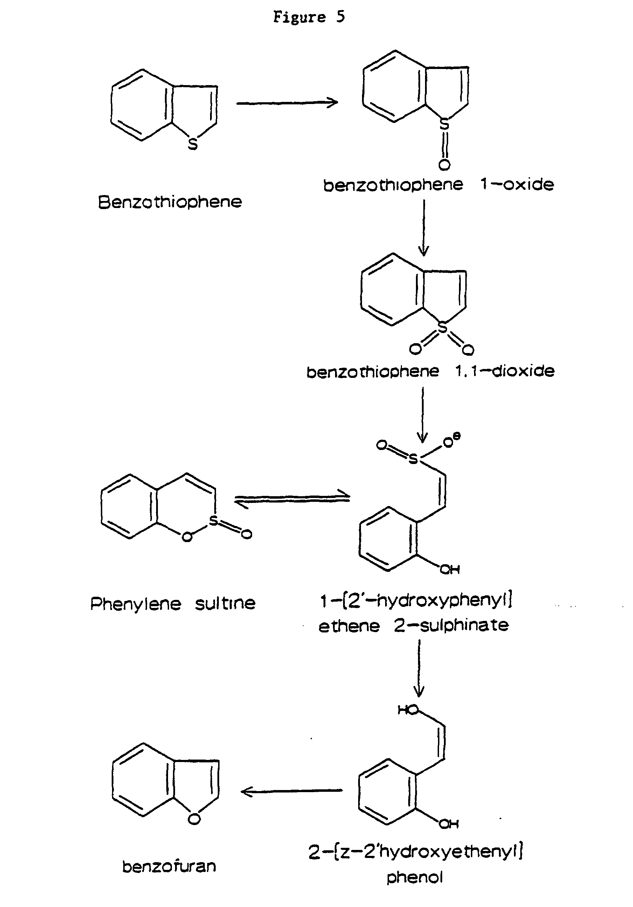

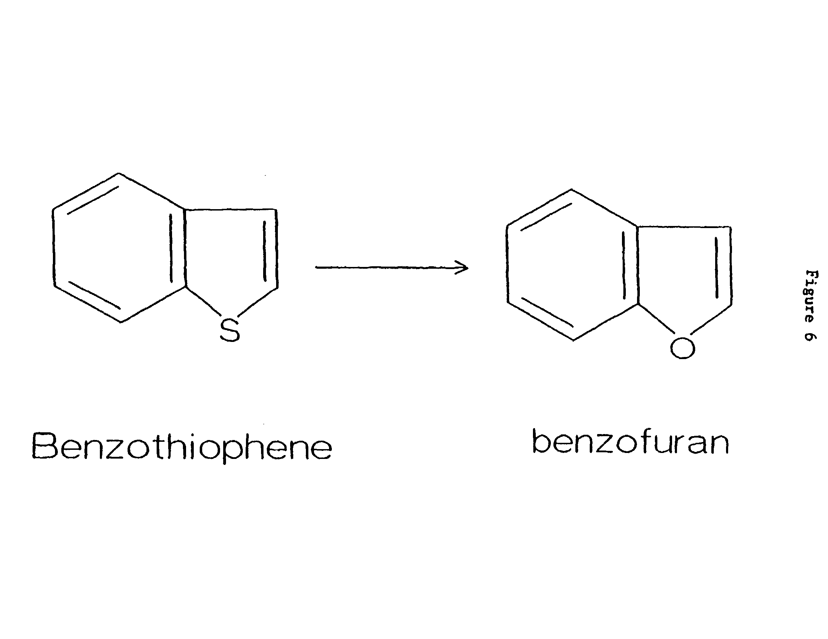

Compounds (I)-(VII) were necessary and sufficient to construct a reasonable metabolic pathway for the desulphurisation of BTH (Fig. 5). The overall reaction results in the conversion of benzothiophene to benzofuran (Fig 6).

Ethyl acetate extracts of strain 213F grown in FruRM/BTH yielded to same set of metabolites as did strain 213E. Furthermore, ethyl acetate extracts of strain 213F grown in FruRM/DBT yielded metabolites common to the DBT desulphurisation pathway of strain IGTS8 (data not shown). Therefore it was concluded that Strain 213F utilised the same BTH desulphurisation pathway as did strain 213E, and the same DBT desulphurisation pathway as did strain IGTS8.

### DISCUSSION

*Rhodococcus* sp. strains 213E and 213F were isolated by enrichment culture in glyRM/BTH (glycerol as sole source of sulphur, BTH as sole source of added sulphur). The background sulphur content of the base medium was kept to a minimum by using glycerol as the carbon source, since this has a lower sulphur content (0.0005 wt% sulphur as sulphate) than does, for example, fructose (0.005 wt%) or glucose (0.0025wt%) (manufacturers' data). In plate media, a very high background of "false positives" was evident in the early stages of the isolation, almost certainly due to the presence of sulphur in the solidifying agent. In preliminary experiments Noble Agar (assayed at 0.889 wt% sulphur as sulphate) was found to give a lower background of false positives, compared with standard Bacto agar, or silica gel. Nevertheless, absolute selection for positives based on plate screening techniques was impossible and all plate colonies were subcultured into liquid GlyRM/BTH media for verification of growth on BTH.

*Rhodococcus* sp. strain 213E grew in a defined minerals medium with a sugar as a carbon source and BTH as sole source of sulphur. During growth a phenolic compound accumulated in the medium, as shown using the Gibbs Phenol assay (Fig 2a). There was no phenol production in cultures of strain 213E grown in SRM/sulphate (data not shown). On the basis of GC-MS analysis of ethyl acetate extracts of the culture medium (Fig-3), the Gibbs-reactive phenol was identified as peak (III), which had a mass-spectrum consistent with its identity as HEP. Since BFU was also present in ethyl acetate extracts it was concluded that HEP condenses to BFU. Effectively, therefore, strain 213E action results in the conversion of HEP to BFU (Figure 6).

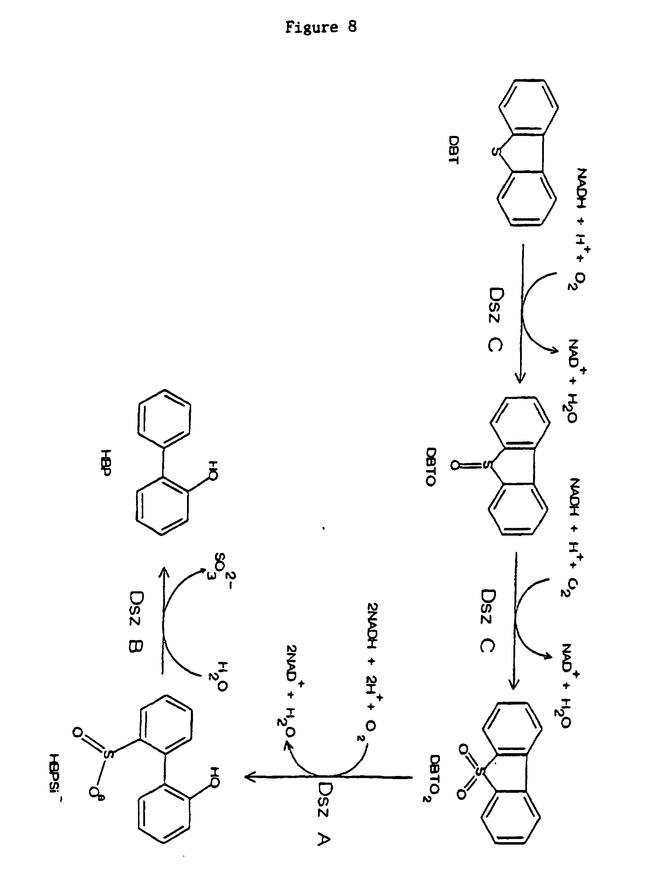

By comparison with the strain IGTS8 DBT desulphurisation pathway (Figure 8, ie Fig 4 in Oldfield et al, 1997) it was straightforward to order the BTH metabolites (Table 3) into the pathway shown in Fig 5. Thus the initial sequence, BTH → BTHO → BTHO₂ mirrors the sequence DBT → DBTO → DBTO₂. The middle step, BTO₂ → HBESi⁻ likewise mirrors the step DBTO₂ → HBPSi. Note that ethyl acetate extraction actually yielded the condensed ("sultine") forms of these sulphinic acids i.e. phenylene sultine (peak IV in Table 3; Fig. 4d), rather than 2-[2'hydroxybenzene] ethen 1-sulphinate (HBESi⁻) and BPSi rather than 2-hydroxybiphenyl 2'-sulphinate (HBPSi⁻; Olson et al, 1993; Oldfield et al, 1997), respectively. However, it has been argued that HBPSi must be the physiologically relevant species on the strain IGTS8 DBT desulphurisation pathway, since BPSi rapidly and completely hydrolyses to HBPSi⁻ in aqueous solution at neutral pH (Oldfield et al, 1997), and for this reason HBESi⁻ is shown as the corresponding step in the BTH desulphurisation pathway (Fig 5). Studies on the Psi ↔ HBESi⁻ equilibrium await the synthesis of these compounds.

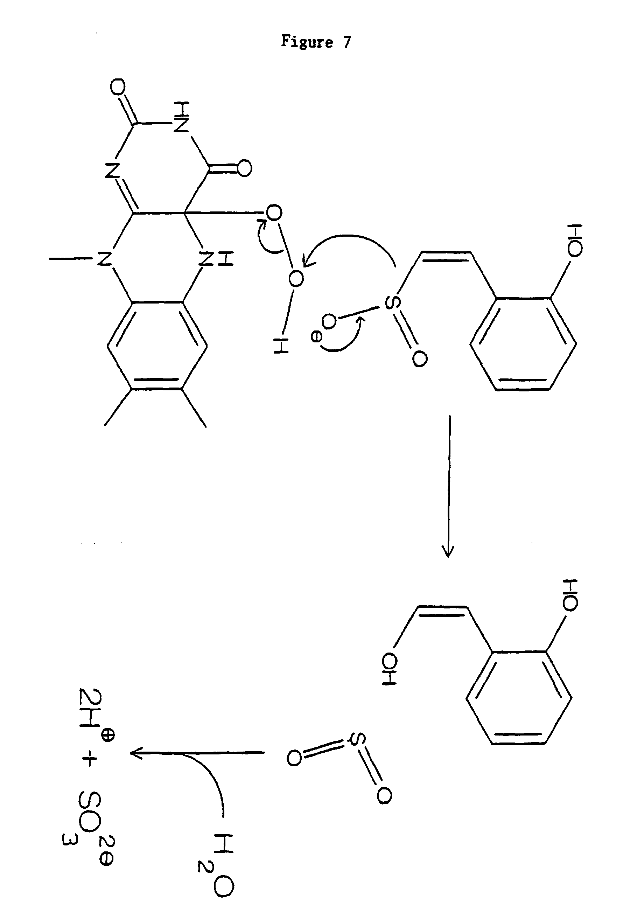

The DBT and BTH desulphurisation pathways diverge at the final step. In the DBT desulphurisation pathway HBPSi⁻ is desulphurised to the monophenol HBP and inorganic sulphite. This reaction is catalysed by Dsz B and is almost certainly an hydrolytic reaction (Oldfield et al, 1997). However, HBESi⁻ is an alkylsulphinic acid and the sulphinate group may not be removed by hydrolysis. Therefore an enzyme equivalent to Dsz B is not expected in the BTH desulphurisation pathway. Since the final desulphurised product is HEP, it seems that removal of the sulphinate group is achieved by a mechanism which results in the hydroxylation of the C-S bond carbon. A reasonable mechanism is shown in Fig. 7 and it is predicted that the final enzyme of the BTH desulphurisation pathway, catalysing HBESi⁻ → HEP is a flavin-dependent hydroxylase catalysing an "oxidative desulphination". Conversion of HEP to BFU is probably a spontaneous (non-catalysed) reaction.

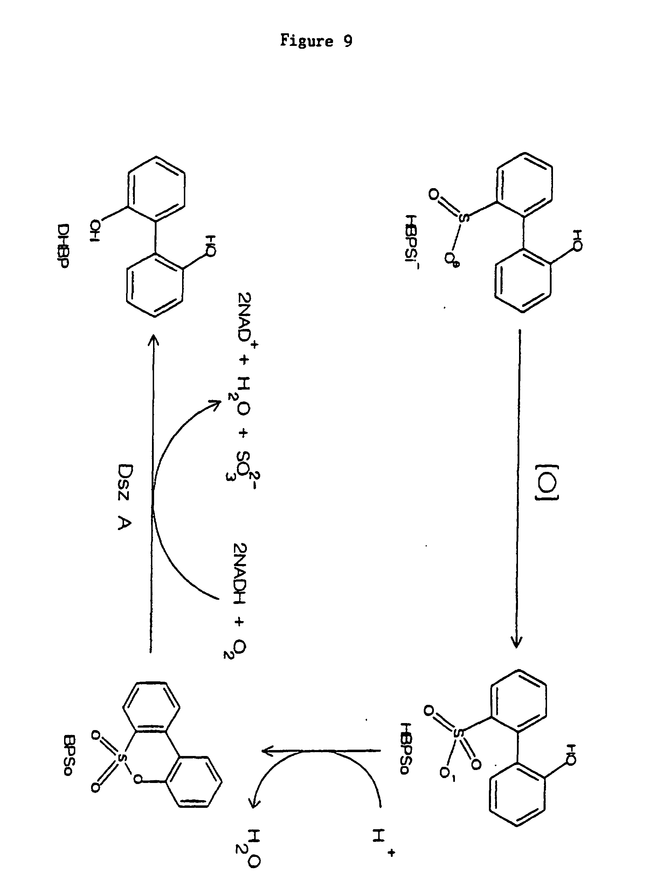

Peak (VII) is a minor peak which has been assigned as phenylene sultone (so). The corresponding species, biphenylene sultone (BPSo) can be isolated from cultures of strain IGTS8 grown on DBT. BPSo is thought to arise by oxidation of HBPSi⁻, to give the corresponding sulphonate, HBPSo, followed by an itramolecular condensation to give BPSo (Oldfield et al 1997). By analogy therefore, the pathway HBESi⁻ → HBESo⁻ → PSi is proposed. In strain IGTS8, BPSo is quantitatively desulphurised to DHBP and sulphite, a reaction is catalysed by Dsz A (Figure 9, ie Fig 6 in Oldfield et al, 1997).

HEP can exist in both cis- and trans- isomeric forms, but this desulphurisation reaction is expected to yield the *cis*-isomer, as shown in Fig 4c and Fig 5, on the basis that the HBESi⁻ desulphination reaction should not disturb the configuration about the alkene C=C double bond. (The mechanism outlined in Fig 7 proceeds with retention of configuration and so is consistent with this assumption). Studies on the isomeric composition of HEP are now in progress.

Strains IGTS8 and 213E have a number of features in common. They both belong to the genus *Rhodococcus* (speciation of strain 213E is currently in progress).
In both cases the function is to release the sulphur moity of the substrate; the carbon skeleton is not metabolised. The pathways do not operate in the presence of more readily bioavailable sulphur and this is consistent with a repression-based regulatory mechanism. If the proposed pathway for BTH desulphurisation is accepted, then it seems that the strategy leading to the cleavage of the first C-S bond, resulting in the opening of the thiophene ring with formation of a sulpinic acid, is identical to that used in the DBT desulphurisation pathway. The pathways must diverge at the last stage however. In the DBT desulphurisation pathway it seems that the sulphinate C-S bond is cleaved by hydrolysis, whereas in BTH desulphurisation both the identity of the product, HEP, and the difficulty of removing an alkyl sulphinic acid group by hydrolysis, suggest that the last step of the BTH pathway is an "oxidative desulphination".

### CONCLUSIONS

This is the first report of an organism which is able to desulphurise benzothiophene. The reaction carried out by *Rhodococcus* sp. strain 213E is analogous to the DBT desulphurisation reaction carried out by *Rhodococcus* sp. strain IGTS8, in the sense that the sulphur moiety is extracted, leaving behind the carbon skeleton, which is not further metabolised. Strain 213E does not desulphurise DBT, and strain IGTS8 does not desulphurise BTH. Therefore, from the point of view of petrochemicals desulphurisation, these organisms are complementary. That the different pathways are required for the two compounds is perhaps self-evident; following through the DBT desulphurisation pathway with BTH yields, at the penultimate step an alkenylsulphinic acid (HBESi⁻) which, in contrast to the arylsulphinic acid, cannot be removed by the simple hydrolytic mechanism proposed for the last enzyme of this pathway, Dsz B. Yet this is not the whole picture, since BTH is not even metabolised as far as the sulphinate by strain IGTS8, and strain 213E does not metabolise DBT. Therefore there seems to be an intrinsic familial 'specificity' built into the desulphurisation pathways.

Further analysis of strain 213F which appears to desulphurise both DBT and BTH may determine this strain as being the most useful organism.

**TABLE 1:**

| Mean generation times and end-log phase biomass for strains IGTS8, 213E and 213F with various sulphur sources at a concentration of 200*µ*M. Strain IGTS8 was grown in SRM, strains 213E and 213F were grown in FruRM. Biomass is reported as the optical density (OD) recorded at 600nm in a 1cm pathlength spectrophotometer cuvette. | | | |
|---|---|---|---|
| **Strain** | **Sulphur Source** | **Mean Generation time/hr** | **Biomass at end log phase/OD**_{**600**} |
| IGTS8 | DBT | 7.0 | 6.0 |
| IGTS8 | sulphate | 3.2 | 7.1 |
| 213E | BTH | 9.0 | 4.2 |
| 213E | sulphate | 4.4 | 5.8 |
| 213F | DBT | 12.4 | 5.4 |
| 213F | BTH | 8.0 | 6.0 |
| 213F | Sulphate | 3.6 | 7.4 |

**TABLE 2:**

| Mean growth rate of Rhodococcus sp. strain 213E in FruRM containing either sulphate or BTH, at different concentrations. | | | |
|---|---|---|---|
| **Sulphur Source** | **Concentration /*µ*M** | **Mean Generation time/hr** | **Biomass at end log phase/OD**_{**600**} |
| sulphate | 200 | 4.4 | 5.8 |
| BT | 50 | 8.0 | 2.3 |
| BT | 100 | 8.6 | 3.0 |
| BT | 200 | 9.0 | 4.2 |

**TABLE 3:**

| Identification of BTH metabolites isolated by ethyl acetate extraction of culture medium (FruRM) used to grow *Rhodococcus* sp. strain 213E grown with BT as sole source of sulphur. | | | | |
|---|---|---|---|---|
| **Peak No** | **GC retention time/min** | **Relative molecular mass** | **Assignment** | **Abbrv.** |
| (I) | 6.7 | 118 | benzofuran | BFU |
| (II) | 9.7 | 134 | benzothiophene | BTH |
| (III) | 10.9 | 136 | 2-[z-2' hydroxyethenyl] phenol | HEP |
| (IV) | 14.0 | 166 | Phenylene sultine | PSi |
| (V) | 14.8 | 150 | benzothiophene 1-oxide | BTHO |
| (VI) | 15.1 | 166 | benzothiophene 1,1-dioxide | BTHO₂ |
| (VII) | 15.3 | 182 | Phenylene sultone | SO |

### Abbreviations

- ACN: acetone
- BFU: benzofuran
- BPSi: biphenylenesultine
- BPSo: biphenylenesultone
- BTH: benzothiophene
- BTHO: benzothiphene 1-oxide
- BTHO₂: benzothiophene 1,1-dioxide
- DBT: dibenzothiophene
- DBTO: dibenzothiophene 5-oxide
- DBTO₂: dibenzothiophene 5,5-dioxide
- DHBP: 2,2'-dihydroxybiphenyl
- FruRM: Fructose Rhodococcus Medium
- GlyRM: Glycerol Rhodococcus Medium
- HBP: 2-hydroxybiphenyl
- HEP: 2-[z-2'hydroxyethenyl] phenol
- HBESi⁻: 2-[2'-hydroxybenzene) ethen 1-sulphinate
- HBESo⁻: 2-[2'-hydroxybenzene] ethen 1-sulphonate
- HBPSi⁻: 2-hydroxybiphenyl 2'-sulphinate
- HBPSo⁻: 2-hydroxybiphenyl 2'-sulphonate
- MGT: mean generation time
- Psi: Phenylene sultine (benzo[c][1,2] oxathiin 6-oxide)
- So: Phenylene sultone (benzo[c][1,2] oxathiin 6,6-dioxide)
- SRM: Standard Rhodococcus Medium

### REFERENCES

**Beutler, H-O.** (1987) Sulfite determination: sulfite oxidase, in Methods in Enzymology 147, 11-14.

**Denis-Larose, C., Labbe, D., Nergeron, H., Jones, A.M. Greer, C.W., Al-Hawari, J., Grossman, M.J., Sankey, B.M. and Lau, P.C.K.** (1997) Conservation of plasmid-encoded dibenzothiophene desulphurisation genes in several rhodococci. App. Env. microbial 63, 2915-2919.

**Denome, S.A., Olson, E.S. and Young, K.D.** (1993) Identification and cloning of genes involved in specific desulphurisation of dibenzothiophene by *Rhodococcus* sp. strain IGTS8 App Env Microbio 59, 2837-2843.

**Denome, S.A., Oldfield, C., Mash, L.J. and Young, K.** (1994) Characterisation of the desulfurization genes from *Rhodococcus* sp. strain IGTS8 J Bacteriol 176, 6707-6716.

**Goodfellow, M.** (1989) Section 26: Nocardioform actinomycetes, genus *Rhodococcus* In: Bergey's Manual of Systematic Bateriology. 2362-2371. Holt, J. G., Ed.

**Gray, K.A., Pogrebinsky, O., Mrashko, G.T., Xi, L., Monticello, D.J. and Squires, C.H.** (1996) Molecular mechanisms of biocatalytic desulphurisation of fossil fuels Nature Biotechnology 14 1705-1708.

**Izumi, Y., Ohshiro, T., Ogino, H., Hine, Y. and Shimao, M.** (1994) Selective desulfurizuation of dibenzothiophene by *Rhodococcus erythropolis* strain D-1 Appl Env Microbiol 60, 223-226.

**Kayser, K.J., Bielaga-Jones, B.A., Jackovski, K., Odusan, O and Kilbane, J.J.** (1993) Utilization of organosulphur compounds by axenic and mixed cultures of Rhodococcus rhodochrous strain IGTS8 J Gen Microbiol 139, 3123-3129.

**Lei, B. and Tu, S-C** (1996) Gene overexpression, purification and identification of a desulfurization enzyme from Rhodococcus sp. strain IGTS8 as a sulfide/sulfoxide monooxygenase J. Bacteriol 178 5699-5705.

**Lee, M.K., Senius, J.D. and Grossman, M.J**. (1995) Sulfur-specific microbial desulfurization of sterically hindered analogs of dibenzothiophene. App. Env. Microbiol. 61, 4362-4366.

**Li, M.Z., Squires, C.H. and Childs, J.D.** (1996) Genetic analysis of the dsz promoter and associated regulatory regions of Rhodococcus *erythropolis* IGTS8 J Bact 178, 6409-6418.

**Oldfield., C., Pogrebinsky, O., Simmonds, J., Olson, E and Kulpa, C.F.** (1997). Elucidation of the metabolic pathway for benzothiophene desulphurisation by *Rhodococcus* sp. strain IGTS8 Microbiology, in press.

**Olson, E.S., Stanley, D.C. and Gallagher, J.R.** (1993) Characterisation of intermediates in the microbial desulfurization of dibenzothiophene Energy & Fuels 7, 159-164.

**Omori, T., Monna, L., Saiki, Y. and Kodama, T.** (1992) Desulfurization of dibenzothiophene by *Corynebacterium* sp. strain SY1 App. Env. Microbiol 58, 911-915.

**Omori, T., Saiki, Y., Kasuga, K. and Kodama, T.** (1995) Desulfurization of alkyl and aromatic sulfides and sulfonates by dibenzothiophene desulphurising *Rhodococcus* sp. strain SY1 Biosci. Biotech. Biochem. 59, 1195-1198.

**Ohshiro, T., Hirata, T. and Izumi, Y. (1996a)** Desulfurization of dibenzothiophene derivatives by whole cells of *Rhodococcus erythropolis* strain H-2. FEMS Microbial Lett 142, 65-70.

**Ohshiro, T., Suzuki, K. and Izumi, Y.** (1996b) Regulation of dibenzothiophene-degrading activity of *Rhodococcus* erythropolis strain D-1. J. Ferm Bioeng 81, 121-124.

**Wang, P. and Krawiec, S.** (1996a) Kinetic analyses of desulfurization of dibenzothiophene by *Rhodococcus erythropolis* in batch and fed-batch cultures App. Env. Microbiol. 62, 3066-3068.

**Wang, P., Humphrey, A.E. and Krawiec, S.** (1996b) Kinetic analyses of desulfurization of dibenzothiophene by *Rhodococcus erythropolis* in continuous cultures App. Env. Microbiol 62. 3066-3068.

## Claims

1. A micro-organism wherein the micro-organism is strain NEU213E deposited under Accession No NCIMB 40816 at the National Collections of Industrial and Marine Bacteria Limited, 23 St Machar Drive, Aberdeen, AB2 1RY on 29 July 1996.

2. A micro-organism wherein the micro-organism is strain NUE213F deposited under Accession No NCIMB 40817 at The National Collections of Industrial and Marine Bacteria Limited, 23 St Machar Drive, Aberdeen, AB2 1RY on 29 July 1996.

3. A micro-organism as claimed in Claim 1 or Claim 2 wherein the micro-organism can desulphurise both benzothiopenes and dibenzothiopenes.

4. A micro-organism as claimed in any of the preceding Claims wherein the micro-organism is rod-shaped and stains Gram positive.

5. A process for the isolation of an organism as claimed in Claim 1 or Claim 2, the process comprising the step of isolating the organism by selective enrichment of micro-organisms capable of utilising benzothiopene as a sole source of sulphur for growth.

6. A process as claimed in Claim 5 wherein the process comprises the steps of obtaining soil samples from the base of an oil shale spoil heap, suspending soil in minerals medium with benzothiopene as sole source of sulphur, repeatedly passaging organisms in the soil in minerals medium containing benzothiopene as sole source of sulphur, streaking cultures on agar plates to obtain isolated colonies of micro-organisms capable of utilising benzothiopene as a sole source of sulphur for growth and preparing cultures from individual colonies.

7. A process as claimed in Claim 5 or 6 wherein the presence of a desulphurising organism is identified when the process accumulates large amounts of phenol in growth medium.

## Patentansprüche

1. Ein Mikroorganismus, wobei der Mikroorganismus Stamm NEU213E ist, der unter der Hinterlegungsnummer NCIMB 40816 bei The National Collections of Industrial and Marine Bacteria Limited, 23 St Machar Drive, Aberdeen, AB2 1 RY am 29. Juli 1996 hinterlegt wurde.

2. Ein Mikroorganismus, wobei der Mikroorganismus Stamm NUE213F ist, der unter der Hinterlegungsnummer NCIMB 40817 bei The National Collections of Industrial and Marine Bacteria Limited, 23 St Machar Drive, Aberdeen, AB2 1RY am 29. Juli 1996 hinterlegt wurde.

3. Mikroorganismus gemäß Anspruch 1 oder Anspruch 2, wobei der Mikroorganismus sowohl Benzothiopene als auch Dibenzothiopene entschwefeln kann.

4. Mikroorganismus gemäß einem der vorhergehenden Ansprüche, wobei der Mikroorganismus stangenförmig ist und grampositiv färbt.

5. Ein Verfahren zum Isolieren eines Organismus gemäß Anspruch 1 oder Anspruch 2, wobei das Verfahren den Schritt des Isolierens des Organismus durch selektive Anreicherung von Mikroorganismen, die Benzothiopen als alleinige Quelle von Schwefel zum Wachstum nutzen können, beinhaltet.

6. Verfahren gemäß Anspruch 5, wobei das Verfahren die Schritte des Erhaltens von Bodenproben aus der Basis einer Ölschieferhalde, des Suspendierens des Bodens in einem Mineralienmedium mit Benzothiopen als alleinige Quelle von Schwefel, des wiederholten Passierens der Organismen in dem Boden in dem Mineralienmedium, das Benzothiopen als alleinige Quelle von Schwefel enthält, des Streifens von Kulturen auf Agar-Platten, um isolierte Kolonien von Mikroorganismen zu erhalten, die Benzothiopen als alleinige Quelle von Schwefel zum Wachstum nutzen können und des Herstellens von Kulturen aus individuellen Kolonien beinhaltet.

7. Verfahren gemäß Anspruch 5 oder 6, wobei das Vorhandensein eines Entschwefelungsorganismus identifiziert wird, wenn das Verfahren große Mengen von Phenol im Wachstumsmedium akkumuliert.

## Revendications

1. Un micro-organisme dans lequel le micro-organisme est la souche NEU213E déposée sous le n° d'accès NCIMB 40816 auprès de la société National Collections of Industrial and Marine Bacteria Limited, 23 St Machar Drive, Aberdeen, AB2 1RY, le 29 juillet 1996.

2. Un micro-organisme dans lequel le micro-organisme est la souche NUE213F déposée sous le n° d'accès NCIMB 40817 auprès de la société National Collections of Industrial and Marine Bacteria Limited, 23 St Machar Drive, Aberdeen, AB2 1 RY, le 29 juillet 1996.

3. Un micro-organisme tel que revendiqué dans la revendication 1 ou la revendication 2 dans lequel le micro-organisme peut désulfurer à la fois les benzothiophènes et les dibenzothiophènes.

4. Un micro-organisme tel que revendiqué dans n'importe lesquelles des revendications précédentes dans lequel le micro-organisme est en forme de canne et se colore Gram positif.

5. Un procédé pour l'isolement d'un organisme tel que revendiqué dans la revendication 1 ou la revendication 2, le procédé comportant l'étape d'isoler l'organisme par l'enrichissement sélectif de micro-organismes capables d'utiliser du benzothiophène comme unique source de soufre pour croître.

6. Un procédé tel que revendiqué dans la revendication 5 dans lequel le procédé comporte les étapes d'obtenir des échantillons de sol de la base d'un terril de schiste bitumineux, de mettre le sol en suspension dans un milieu de minéraux avec du benzothiophène comme unique source de soufre, de faire passer à plusieurs reprises des organismes dans le sol dans le milieu de minéraux contenant du benzothiophène comme unique source de soufre, de déposer des cultures en stries sur des plaques de gélose pour obtenir des colonies isolées de micro-organismes capables d'utiliser du benzothiophène comme unique source de soufre pour croître et de préparer des cultures à partir de colonies individuelles.

7. Un procédé tel que revendiqué dans la revendication 5 ou la revendication 6 dans lequel la présence d'un organisme désulfurant est identifiée quand le procédé accumule de grandes quantités de phénol dans du milieu de croissance.
